**Europäisches Patentamt**

**European Patent Office**

**Office européen des brevets**

(19)

(11) Publication number : **0 035 204 B2**

(12) # NEW EUROPEAN PATENT SPECIFICATION

(45) Date of publication of the new patent specification : **22.05.91 Bulletin 91/21**

(21) Application number : **81101294.7**

(22) Date of filing : **23.02.81**

(51) Int. Cl.⁵ : **A61L 2/04**, A61K 37/02, A61K 39/395, A61K 37/24, A61K 37/64, A61K 37/54, A61K 37/04

(54) Pasteurized therapeutically active protein compositions.

(30) Priority : **05.03.80 US 127351**
**31.10.80 US 202508**

(43) Date of publication of application :
**09.09.81 Bulletin 81/36**

(45) Publication of the grant of the patent :
**21.10.87 Bulletin 87/43**

(45) Mention of the opposition decision :
**22.05.91 Bulletin 91/21**

(84) Designated Contracting States :
**AT BE CH DE FR GB IT LI NL SE**

(56) References cited :
EP-A- 0 018 561
FR-A- 2 460 138
GB-A- 1 230 127
GB-A- 2 000 956
US-A- 2 897 123
US-A- 3 227 626
US-A- 3 293 236
US-A- 3 301 842
US-A- 3 903 262
US-A- 4 046 877
US-A- 4 061 735
US-A- 4 089 944

(56) References cited :
US-A- 4 137 307
US-A- 4 160 025
US-A- 4 178 368
US-A- 4 186 192
JOURNAL OF THE AMERICAN CHEMICAL SOCIETY, vol. 75, no. 21, 11th November 1953, pages 5139-5152, R.B. SIMPSON et al.: "The kinetics of protein denaturation. I. The behavior of the optical rotation of ovalbumin in urea solutions"
JP-A-33369/1981 (with English translation)
JP-A-118819/1970 (with English translation)
Biochemistry, Vol. 18, No. 23, pp. 5191-5196 (1979)

(73) Proprietor : **MILES INC.**
**1127 Myrtle Street**
**Elkhart Indiana 46514 (US)**

(72) Inventor : **Fernandes, Peter M.**
**4405 Corkwood Ct.**
**Concord California 94521 (US)**
Inventor : **Lundblad, John L.**
**1390 Club View Ct.**
**El Cerrito California 94530 (US)**

(74) Representative : **Adrian, Albert, Dr. et al**
**c/o BAYER AG Konzernverwaltung RP**
**Patentabteilung**
**W-5090 Leverkusen 1, Bayerwerk (DE)**

EP 0 035 204 B2

## Description

This invention relates to a method for pasteurizing a composition containing a thermally sensitive, therapeutically active protein selected from, antithrombin-III, prekallikrein, antihemophilic factor (factor VIII) and fibronectin capable of being stabilized during pasteurisation in the presence of a polyol as the only stabilizer which is a water miscible polyhydric alcohol or cabohydrate having a molecular weight less than about 5000, which comprises

a) mixing about one part of protein composition with 4 to 200 parts of an aqueous polyol having a concentration of said polyol ranging from 30% (w/v) to saturation and

b) heating the mixture at a temperature of 60 to 75°C at a pH of 5.5 to 8.0 for about 10 hours.

Description of the prior art

Many useful blood fractions and blood proteins are obtained from human blood plasma by fractionation according to known techniques such as, for example, the alcohol fractionation method of Cohn described in U.S. Patent No. 2,390,074 (1945) and the *Journal of the American Chemical Society*, Vol. 68, page 459 (1946) and the Rivanol® ammonium sulfate method. The aforementioned methods as well as other variations and techniques are summarized in "The Plasma Proteins", second edition, Volume III, pages 548-550, Academic Press, New York, New York (1977). These blood fractions contain biologically active proteins that possess certain therapeutic qualities. For instance, Factor VIII or antihemophilic factor is useful against hemophilia ; plasminogen is a precursor of plasmin for treatment of acute thromboembolic disorders ; immune serum globulin (IgG) is employed in the treatment of congenital gamma globulin deficiency, measles, poliomyelitis and hepatitis A and B ; fibronectin has been identified as active in treatment of burns, shock, cancer, etc. ; antithrombin III is a coagulation inhibitor, cryoprecipitate itself may be used directly for classic hemophilia ; Plasma Protein Fraction (human) and albumin are useful in treatment of shock due to burns, crushing injuries, abdominal emergencies, and any other cause producing a predominant loss of plasma fluids and not red cells ; immune globulin, intravenous (modified immune serum globulin) is a substitute for immune serum globulin administrable in larger quantities ; Factor VIII inhibitor bypassing active (FEIBA) substance described in U.S. Patent 4,160,025 as a blood-coagulation-promoting preparation for Factor VIII inhibitor patients ; $\alpha$-1-antitrypsin can be employed in the treatment of emphysema ; plasma growth hormone corrects pituitary growth deficiency, somatomedin is useful in correcting growth deficiencies, other immune serum globulins, e.g., IgA, IgD, IgE, and IgM, may be employed to treat various immune protein deficiencies ; prealbumin (U.S. Patent 4,046,877) is employed to increase immunologic competence ; plasminogen-streptokinase complex (U.S. Patent 4,178,368) can be administered to patients for treatment of thromboembolisms ; ceruloplasmin, transferrin, haptoglobin, and prekallikrein have reagent and other uses.

One problem confronting users of plasma, plasma fractions, and compositions containing individual blood proteins is the thermal instability of the therapeutically active proteins contained therein. In many cases, substantial, and sometimes complete, losses of activity are observed if these proteins are heated above physiological temperatures, i.e., above about 40-45°C. Consequently, these items require special care during preparation and storage to minimize such deactivation.

The thermal instability of the aforementioned proteins renders them unpasteurizable. Therapeutically active proteins isolated from plasma may contain viruses, e.g., hepatitis virus, present in the source material for the protein fraction, namely, blood from a donor. A risk of contracting hepatitis exists, therefore, for those receiving unpasteurized fractions from blood plasma fractionation because the presence of the virus cannot be detected with certainty by any known procedure. In a large number of situations, this risk is outweighed by the detriment to a patient in not receiving the therapeutic plasma fraction as determined by the physician.

Some therapeutically active proteins derived from plasma have been pasteurized successfully. For example, it is well known that albumin can be pasteurized by heating at 60°C or 64°C for 10 hours (Gellis *et al., J. Clin. Invest.*, Vol. 27, pages 239-244 (1948)) in the presence of certain stabilizers such as acetyl-tryptophan and sodium caprylate. Individuals receiving this pasteurized material did not contract hepatitis, thus indicating the inactivation of hepatitis virus while retaining the activity of albumin under the afore-described heating conditions. Plasma Protein Fraction (human) is also stabilized during pasteurization by the above method.

A process for pasteurizing plasminogen is disclosed by Baumgarten et al. in U.S. Patent 3,227,626. An aqueous preparation containing 0.25-20 milligrams per milliliter (mg/ml) of plasminogen and further containing 0.1-0.5 molar lysine with a pH of 5.3-7.5 was heated at 60°C for 10 hours. As the patentee states, hepatitis virus was destroyed and the danger of transmitting hepatitis was removed with retention of plasminogen activity. Attempts to pasteurize plasminogen under the above conditions in the absence of lysine resulted in complete destruction of plasminogen activity. It is interesting to note that plasminogen cannot be stabilized with N-acetyl-tryptophan and sodium caprylate during pasteurization, nor can albumin and Plasma Protein Fraction (human) be pasteurized in the presence of lysine.

Singher has described a process for treating plasminogen to produce a material that is not contaminated with hepatitis virus (U.S. Patent 2,897,123). In the patented pasteurization technique aqueous solutions of plasminogen are heated at about 60°C for about 10 hours. The activity of plasminogen is retained if the solutions have a pH in the range not less than 3 nor greater than 6.5 and an ionic strength not greater than 0.3.

Another method for removing hepatitis virus from a biological material is described in U.S. Patent 4,168,300. The material to be treated is contacted with a preparation, which may be agarose gel or beaded polyacrylamide plastic coupled with a variety of hydrophobic ligands. Plasma and albumin were subjected to the above purification technique to remove hepatitis virus.

Aqueous solutions of the enzyme thrombin have been stabilized (Seegers, *Arch. Biochem., 1944*. Vol. 3, pages 363-367) during heating at 50°C in the presence of saturation amounts of certain glycosides. The stabilized solutions were heated at the above temperature for a period of 48 hours or more with minimal loss of activity. On the other hand, Seegers also discloses that glycosides and polyols have only minimal effectiveness in stabilizing the enzyme prothrombin. The reversible denaturation of lysozyme and ribonuclease was studied by Gerlsma *et al., Int. J. Peptide Protein Res.*, Vol. 4, pages 377-383 (1972). The authors found that certain polyhydric alcohols increased somewhat the temperatures at which these enzymes were denatured. Finally, Simpson et al., in *J. Am. Chem. Soc.*, Vol. 75, No. 21, pages 5139-5152 (1953) and Donovan in *J. Sci. Fd. Agric.*, Vol. 28, pages 571-578 (1977) noted that the denaturation temperature of ovalbumin (an egg white protein) was raised slightly in the presence of sucrose in aqueous solutions of the protein. However, Donovan points out that the temperatures of denaturation of ovalbumin and S-ovalbumin are 84.5°C and 92.5°C, respectively. Furthermore, ovalbumin and S-ovalbumin, as well as the aforementioned enzymes, have no therapeutic activity in treating disorders in humans, whereas blood plasma proteins are therapeutically active. In fact, as mentioned below, proteolytic enzymes deactivate blood plasma proteins.

Singher, in the aforementioned U.S. Patent, lists some methods of destroying hepatitis virus. The least effective of these methods involves the use of either nitrogen mustard or β-propiolactone. High energy irradiation in appropriate dosage is effective but destroys biological activity when applied to human blood products. Heat is recognized also as effective against hepatitis virus, the preferred treatment being heating the material at 60°C for 10 hours. Higher temperatures above 70°C for shorter intervals or lower temperatures for longer intervals have also been tried with successful results. However, it is important to note that higher temperatures are undesirable because of the potential for denaturation of the proteins. Furthermore, lower temperatures for long intervals are to be avoided because various proteolytic enzymes are activated under these conditions, and these activated enzymes cause protein degradation. Also, the use of temperatures lower than 60°C for pasteurization has not been shown to consistently yield a material that does not contain the infective virus.

As mentioned above, the recognition that heating at 60°C and 64°C for 10 hours successfully destroys the hepatitis virus in albumin was made by Gellis *et al., supra. Gellis et al.* proved experimentally that albumin heated under the above conditions did not transmit hepatitis even if hepatitis virus was present prior to pasteurization. However, the author noted that hepatitis virus survived heating at 56°C for one hour, a temperature usually employed for the inactivation of viruses. Thus, although heating at temperatures of about 56°C for one hour will deactivate most viruses, hepatitis virus is not inactivated ; and materials containing hepatitis virus, which are heated at 56°C for one hour, cause infection of hepatitis in individuals receiving such materials.

In German Offenlegungsschrift No. 29 16 711 which is no prior publication to the instant invention, a method is described for heat-stabilisation of aqueous solutions of plasminogen, antihemophilic factors II, VIII and XIII and antithrombin III by adding an amino acid and a monosaccharide, oligosaccharide or sugar alcohol to the solutions.

## Summary of the invention

The invention described herein provides a method for pasteurizing a composition contianing a thermally sensitive, therapeutically active protein selected from antihrombin-III prekallikrein, antihemophilic factor (factor VIII) and fibronectin capable of being stabilized during pasteurisation in the presence of a polyol as the only stabilizer which is a water miscible polyhydric alcohol or cabohydrate having a molecular weight less than about 5000, which comprises

a) mixing about one part of protein composition with 4 to 200 part of an aqueous polyol having concentration of said polyol ranging from 30% (w/v) to saturation and

b) heating the mixture at a temperature of 60 to 75°C at a pH of 5.5 to 8.0 for about 10 hours.

The primary advantage of the invention is the availability of thermally stable and pasteurized therapeutically active protein compositions, which heretofore have been unknown and unattainable. Since the therapeutically active protein compositions of the invention can be heated with minimal loss of activity under conditions known to inactivate hepatitis virus, these valuable materials can be administered to patients, who can obtain the full therapeutic benefits thereof with a substantially reduced risk of being infected by the hepatitis virus.

Another advantage of the invention is that it may be applied to blood plasma prior to fractionation, to partially fractionated blood plasma, and to individual blood plasma fractions, as well as to individual blood plasma proteins themselves. Thus, the versatility of the present process can be seen. Pasteurization of blood plasma prior to fractionation allows fractionation techniques other than the Cohn process to be applied to whole plasma. In this fashion the yield of albumin can be increased by about 20% or more. To date the Cohn process has been relied upon to secure certain therapeutic fractions such as immune serum globulin which without pasteurization have a history of being non-hepatitis infective. It must be remembered that immune serum globulin has not been pasteurized prior to this invention.

Description of the preferred embodiments

As mentioned above, the products obtainable according to the invention include pasteurized or heat-treated compositions comprising a thermally sensitive, therapeutically active protein capable of being stabilized during pasteurization or heating at temperatures of about 60-75°C, preferably about 60-70°C when mixed with thermal-stabilizing or pasteurization-stabilizing amounts of a polyol, the pasteurized compositions containing or being free of polyol.

In the method of the invention, the protein composition to be pasteurized is suspended or dissolved in an aqueous medium with an amount of polyol sufficient to stabilize the protein composition during subsequent pasteurization. The concentration of polyol necessary to stabilize a protein composition in accordance with this invention depends on the type and concentration of therapeutically active protein in the protein composition and on the type of polyol itself. Generally, the thermal-stabilizing amount or pasteurization-stabilizing amount should be within the range of about 1-1000 parts, preferably 5-100 parts, of polyol per part of total protein in the protein composition. Generally, about one part of protein composition is mixed with 4-200 parts, of an aqueous medium containing at least about 30%, to saturation, preferably at the temperature of pasteurization of polyol, on a weight to volume basis. The therapeutically active protein is considered to be stabilized if it retains a substantial portion, i.e., at least 40%, of its therapeutic activity during pasteurization. It is preferred that 70% or more of the therapeutic activity of the protein composition be retained during pasteurization. Consequently, the amount of polyol to be added should be such as to retain the above-recited amount of therapeutic activity.

After the protein composition has been mixed with the polyol, the mixture is heated at a temperature and for a time sufficient to pasteurize it. Thus, the mixture is pasteurized upon heating it under conditions known to inactivate hepatitis virus. Effective pasteurization to inactivate hepatitis virus and to substantially reduce the risk of hepatitis infection is obtained by heating an unpasteurized protein composition at a temperature about 62-65°C for about 10 hours.

The pasteurization is carried out under pH conditions which approximate physiological conditions. Thus, the pH of the mixture usually should be within the range of about 6.0-7.5.

In general, physiological conditions are desirable, where possible, during pasteurization to insure the least disturbance to the therapeutically active protein composition.

The amount of a particular polyol required to stabilize a specific protein composition during pasteurization and the conditions necessary to pasteurize the composition can be determined readily by one skilled in the art using pilot trials in accordance with the teaching contained herein.

Following pasteurization the mixture of polyol and protein composition may be treated to remove all or part of the polyol. Conventional techniques can be employed to achieve this end. For example, the mixture can be dialyzed or diafiltered using an appropriate semi-permeable membrane. Other means of removing the polyol will be suggested to those skilled in the art.

The pasteurized mixture may be treated to remove water therefrom by procedures well known in the art. For instance, the mixture can be freeze-dried or ultrafiltered and then freeze-dried. Furthermore, the mixture can be sterile-filtered by conventional methods prior to water removal.

The pasteurized protein compositions of the invention can be formulated into pharmaceutical preparations for therapeutic use. To prepare it for intravenous administration the protein composition is dissolved usually in water containing physiological substances such as sodium chloride and glycine and having a buffered pH compatible with physiological conditions. Generally, guidelines for intravenously administered protein compositions are established by governmental regulation.

Thermally sensitive, therapeutically active proteins included within the scope of the invention are those proteins generally administered to patients for preventative and/or curative purposes, which lose some therapeutic activity when heated above about 40-45°C and which are capable of being stabilized during pasteurization or heating at a temperature of about 60-75°C in the presence of a polyol. The therapeutically active proteins that may be pasteurized in accordance with the present invention, antihemophilic factor (Factor VIII), fibronectin (cold insoluble globulin), antithrombin III, and prekallikrein. The pasteurized defatted compositions can be administered to patients who cannot tolerate infusion of high fatty acid material such as that obtained using standard pasteurization stabilizing agents, namely, sodium caprylate and sodium acetyl-tryptophanate.

It is noteworthy that antihemophilic factor B (Factor IX) and prekallikrein activator cannot be pasteurized in the presence of a polyol in accordance with the above method. Indeed, these proteins lose substantially all their therapeutic activity under conditions under which the aforementioned protein compositions retain a substantial portion of their activity.

The term "polyol" means a substance with more than one hydroxyl group (–OH) and includes polyhydric alcohols and carbohydrates such as sugars. It is preferred that the polyol be water miscible, physiologically compatible with the protein, and have a low molecular weight, i.e., a molecular weight less than about 5000. Higher molecular weight polyols, e.g., polysaccharides such as dextrin, starch, glycogen, cellulose, pentosans, pectin and hemicellulose, are not preferred for use in the present method because they are generally water immiscible and are difficult to separate from the protein composition after pasteurization has been completed.

Typical examples of sugars that may be employed in our method are mono-. di-, and trisaccharides such as arabinose, glucose, galactose, fructose, ribose, mannose, rhamnose, sucrose, maltose, raffinose and melezitose. Exemplary of polyhydric alcohols or reduced sugars, included within the purview of the invention are erythritol, ribitol, sylitol, sorbitol and mannitol.

Also within the compass of the invention are mixtures of polyols and substances that produce a polyol in the presence of water or heat such as hydrates and actonides.

It has been found that the fibrinogen (Factor I) content of the protein composition to be pasteurized is an important factor, the higher the fibrinogen content, the greater the amount of carbohydrate needed. The fibrinogen content of the protein composition should be no greater than about 60%, based on the weight of total protein, or no greater than 0.6% based on the weight of solution at, for example, 54% sucrose (weight to volume). In a preferred embodiment the protein composition should contain no greater than 40% fibrinogen, based on the weight of solution. If the amount of fibrinogen in the composition to be pasteurized exceeds the above limits and the amount of carbohydrate is not increased, the thermal stability imparted to the therapeutically active proteins by the polyol is substantially reduced or lost completely.

A protein composition having a fibrinogen content greater than 60% can be pasteurized in accordance with our method (1) if the concentration of fibrinogen in the solution is below 0.6%, preferably below 0.4% or (2) if a protein capable of being stabilized during pasteurization such as albumin is first added to the protein composition to lower its fibrinogen content to less than 60% (the added protein generally should have the characteristic of being easily separable from the initial protein composition if necessary ; it may be also that the added protein is compatible with the intended therapeutic use of the initial protein composition and, thus, need not be removed therefrom), or (3) if at least about 5 parts of carbohydrate are used per part of the fibrinogen.

Important products of this invention include pasteurized aqueous mixtures of polyol and therapeutically active protein compositions. Also included within the scope of this invention are pasteurized compositions free of polyol but containing a therapeutically active protein and those pasteurized protein compositions being free of polyol and water. Pharmaceutical preparations containing therapeutic amounts of a protein composition pasteurized in accordance with the present invention are also contemplated. Particular products of the invention are pasteurized compositions containing therapeutically active proteins that have not been pasteurized prior to this invention. The products of the invention include pasteurized compositions containing antihemophilic factor (Factor VIII), fibronectin.

As mentioned above the pasteurized products of the invention may be incorporated into pharmaceutical preparations, which may be used for therapeutic purposes. However, the term "pharmaceutical preparation" is intended in a broader sense herein to include preparations containing a protein composition pasteurized in accordance with this invention used not only for therapeutic purposes, but also for reagent purposes as known in the art ; for tissue culture wherein organisms such as viruses for the production of vaccines, and interferon, are grown on plasma or on plasma fractions, e.g., Cohn Effluent II + III, Cohn Fraction IV, and Cohn Fraction V.

For any of the above uses it is advantageous that the protein composition be free of infective hepatitis as provided in the instant invention. The pharmaceutical preparation intended for therapeutic use should contain a therapeutic amount of a pasteurized protein composition, i.e., that amount necessary for preventative or curative health measures. If the pharmaceutical preparation is to be employed as a reagent, then it should contain reagent amounts of pasteurized protein composition.

Similarly, when used in tissue culture or a culture medium the pasteurized protein composition should contain an amount of protein composition sufficient to obtain the desired growth. It should be obvious that protein compositions pasteurized in accordance with this invention will not contain infective amounts of viruses and other ogranisms which are inactivated under the pasteurization conditions.

The invention described above is demonstrated further by the following illustrative examples.

Example 1

Pasteurization of antihemophilic factor

A. Four vials of Koate® antihemophilic factor (Factor VIII produced by Cutter Laboratories, Inc.. Berkely, California) were reconstituted in 10 ml of water-for-injection (WFI) each (1.9 mg of protein/ml). The vials were pooled and treated with sucrose (0.8 g/ml). The mixture was heated at 60°C for 10 hours to pasteurize it and then dialyzed overnight against final container AHF buffer (0.3 M glycine, 0.15 M sodium chloride, 0.01 M sodium citrate, and 1% dextrose).

As a control another vial of Koate® antihemophilic factor was reconstituted with 10 ml WFI and saturated with sucrose. The sample was stored at 5°C and was not heated at 60°C for 10 hours. The stored sample was dialyzed as described in the preceding paragraph.

The pasteurized sample and the control from above were assayed as follows :

Total protein was determined by absorbancy measurements at 280 nanometers.

Procoagulant activity (VIII : C) was assayed by one stage Activated Partial Thromboplastin Time (APTT) test modified from the methods of Langdell et al., J. Lab. Clin. Med., Vol. 41, pages 637-647 (1953) and Proctor et al., Am. J. C/in. Path., Vol. 36, page 212 (1961).

Ristocetin-Willebrand factor activity (VIII R : WF) was assayed with gel-filtered platelets according to the method of Olson et al., Am. J. Clin. Path., Vol. 63, pages 210-218 (1975).

Quantitative factor VIII antigen (VIII R : Ag) determinations were done according to the procedure of Laurell, Anal. Biochem., Vol. 15, pages 45-52 (1966).

Antiserum against the Factor VIII related proteins was obtained from Behring Diagnostics (Sommerville, New Jersey).

Protein species distribution was assayed by cellulose acetate electrophoresis.

The results are summarized in the table below.

TABLE 1

| Sample | VIII:C/ml (units) | $A_{280}$ (units) | Recovery of VIII:C (%)[a] |
|---|---|---|---|
| Pasteurized | 2.0 | 2.5 | 63[b] |
| Control | 3.1 | 3.6 | 66[b] |
| Feed | 22.2 | 17 | — |

[a] $$\frac{\text{units VIII:C/units } A_{280}}{\text{units VIII:C (feed)/units } A_{280} \text{ (feed)}} \times 100$$

[b] These results indicate that the loss in AHF activity observed results from procedures other than the pasteurization step, such as the dialysis procedure.

B. AHF concentrate was prepared by a modified method of Hershgold et al., J. Lab. and Clin. Med., Vol. 67, pages 23-32 (1966) from fresh frozen human plasma.

To an aqueous solution of AHF concentrate was added enough sodium citrate and sodium chloride to achieve concentrations of 0.01 M and 0.15 M, respectively, and glycine was added to a concentration of 1.6 M. The mixture was held at 5°C for 1 hour. The precipitate that formed was collected by centrifugation and removed. The solution was dialyzed against 0.1 M glycine, 0.01 M citrate, and0.15 M sodium chloride using an Amicon® hollow fiber system (Amicon Corp., Bedford, Massachusetts) to reach final container levels of the above materials.

The AHF solution was concentrated by means of ultrafiltration using an AmiconPM10 membrane to an $A_{280}$ of 20. Following this step, the AHF solution was mixed with enough sucrose to yield a sucrose solution containing (0.8 g/ml). The mixture was heated at 60°C for 10 hours to pasteurize it and then dialyzed against final container AHF buffer.

The pasteurized AHF concentrate was analyzed according to the procedures enumerated in Part A of this Example. The results are tabularized in Table 2.

TABLE 2

| Sample | Vol. (l) | $A_{280}$ | VIII:C (u/ml) | VIIIR:WF (u/ml) | VIIIR:Ag (u/ml) | VIIIR:Ag / VIIIR:WF | Recovery | |
|---|---|---|---|---|---|---|---|---|
| | | | | | | | VIII:C (%) | VIIIR:WF (%) |
| Pasteurized | 2.89 | 5 | 1.1 | 1.6 | 1.8 | 1.6 | 66 | 61 |
| Feed solution | 1.93 | 8.1 | 3.6 | 3.0 | 8.8 | 2.9 | 100 | 100 |

Example 2

Pasteurization of prekallikrein

Prekallikrein was prepared as follows : Human plasma was dialyzed against 0.05 M Tris, 0.2% Polybren® solution at pH 8.0 to lower its ionic strength. The dialyzed plasma was then applied to a DEAE Sephadex® A50 column and fractionated. The active prekallikrein peaks were pooled and then contacted with Concanavalin A-Sepharose®. The Concanvalin A-Sepharose was washed, and prekallikrein was eluted with 0.5 M $\alpha$-methyl-mannoside. The prekallikrein enriched solution (0.5 mg protein/ml) was dialyzed and sterile filtered. Five milliliters of prekallikrein solution was mixed with sucrose (0.8 g/ml) and heated at 60°C for 10 hours. The recovery of prekallikrein activity was determined by first activating it to kallikrein using excess prekallikrein activator (PKA or Factor XIIa). The resulting kallikrein was used to cleave standardized amounts of benzoyl arginine ethyl ester (BAEE). The extent of cleavage was monitored spectrophotometrically at 253 nanometers (nm). The recovery of prekallikrein activity was 85%.

As a control one ml of prekallikrein solution without sucrose was heated at 60°C. Total activity was lost within three hours ; no visual precipitation was observed.

Example 3

Pasteurization of fibronectin

Fibronectin was prepared by the method described by Engvall et al., Int. J. Cancer, Vol. 20, page 2 (1977) by the gelatin-Sepharose® affinity medium method. Fibronectin was eluted from the affinity medium by washing with 4M urea.

To remove urea the fibronectin-urea eluate was diafiltered using an Amicon® hollow fiber membrane cartridge with a nominal retention of 10,000 molecular weight against Buffer A in 10% sucrose (0.1 g/ml).

The retentate was analyzed for fibronectin content by gel electrophoresis on unreduced and reduced sodium dodecylsulfate (SDS) polyacrylamide gel. The fibronectin band accounted for greater than 90% of the protein visible on the gel. The molecular weight of the fibronectin on unreduced SDS polyacrylamide gel was 400,000-500,000 ; the repoted molecular weight is 450,000-500,000 by Yamada et al., Nature, 1978, Vol. 275, page 179. The molecular weight of the fibronectin on reduced SDS polyacrylamide gel was 250,000-270,000; the reported value is 210,000-250,000 by Yamada et al., supra.

The fibronectin was identified further by its amino acid composition, which agreed with that reported by Yamada et al., Biochemistry, 1977, Vol. 16, page 5552.

An antibody to the above-prepared purified fibronectin was prepared and it cross-reacted with commercially available fibronectin (Collaboration Research, Inc., Waltham, Massachusetts). In immunodiffusion studies the above-prepared fibronectin showed a line of identity with the commercial fibronectin when both samples were cross-reacted against the above antibody.

The above-prepared fibronectin was demonstrated to be greater than 95% pure as determined by stained SDS polyacrylamide gels.

Run A. The retentate (3.6 l) was mixed with additional sucrose such that the final concentration of sucrose was 57% (0.81 g/ml). The mixture was pasteurized by heating at 60°C for 10 hours, was cooled, and was diafiltered against Buffer A (several volume exchanges) using an Amicon® cartridge as described above to remove sucrose. Then, the solution was sterile filtered in Buffer A (0.05 M $Na_3PO_4$ buffer (pH 7.6) and 0.1 M NaCl).

The activity of the pasteurized fibronectin (Run A) was determined by the following assays :

Rat liver slice assay described by Molnar *et al.* in Biochemistry, Vol. 18, page 3909 (1979). The assay was performed in scintillation vials with 12 to 16 µg of added fibronectin, 10 units of heparin, gelatin-coated latex particles labelled with [125]I(10,000 cpm added to each vial), Krebs-Ringer buffer to a final volume of 1.2 ml, and a 100-150 mg slice of fresh rat liver. This mixture was incubated for 30 minutes at 30-37°C with shaking. Uptake of labelled gelatin-latex, was enhanced up to 10 fold by native fibronectin and by sucrose-pasteurized fibronectin, but not by fibronectin which was heated at 60°C for more than 30 minutes in the absence of sucrose.

Agglutination assay described by Check *et al.* in the *J. Reticuloendothelial Soc.*, Vol. 25, pages 351-362 (1979). The assay was performed in a manner similar to the liver slice assay. Fibronectin (2 to 31 µg, sometimes up to 600 µg) was added to vials containing 10 units of heparin,300 µl of a 0.6% solution of unlabelled gelatin-coated latex, and Krebs-Ringer buffer to a final volume of 1.2 ml. Vials were shaken for 30 minutes at 30-37°C. Agglutination was scored visually by noting a transition from a milky solution to a clear solution with clumped particles. Activities are expressed as the lowest added amount of fibronectin at which agglutination occurs, and sucrose-pasteurized fibronectins caused visible agglutination of gelatinlatex particles at 8-23 µg of added fibronectin/vial. Fibronectin heated at 60°C for more than 30 minutes in the absence of sucrose had to be added at levels more than 570 µg/vial to show agglutination, indicating a considerable loss of activity.

Run B. The experiment of Run A was repeated with the following addition : retentate after mixing with sucrose, was combined with arginine to a final concentration of arginine of 0.5 molar.

Run C. The experiment of Run B was repeated but no sucrose was added to retentate (not in accordance with the invention).

Run D. The experiment of Run C was repeated with 0.5 molar lysine in place of the arginine (not in accordance with the invention).

Run. E. As a control retentate (3.6 l) was heated at 60°C for 10 hours, and was cooled, diafiltered and sterile filtered as described in Run A.

Run F. As a control retenate (3.6 l) was constituted with sucrose to a concentration of 57% as in Run A and held at 5°C for 10 hours. This sample was not heated.

Samples from Runs B-E were analyzed by the aforementioned assays. The results from Runs A-E are outlined in the following table.

TABLE 3

| Run | Pasteurization conditions (60°C, 10 hours) | Recovery of therapeutic activity | |
| --- | --- | --- | --- |
| | | Agglutination assay (%) | Rat liver slice assay (%) |
| A | 57% sucrose | 105 | 120 |
| B | 57% sucrose, 0.5 M arginine | 118 | 60 |
| C[a] | 0.5 M arginine | 10 | — |
| D[a] | 0.5 M lysine | 5 | — |
| E[a] | control | <1 | 0 |
| F[a] | control, held at 5°C for 10 hours | 100 | 100 |

[a] Not in accordance with the invention but provided for purposes of comparison.

The state of the pasteurized fibronectin was assessed by two additional assays :

Electrophoresis in non-denaturing buffers on polyacrylamide gels (Peacock, A. C., *et al.*, Science *147*, 1451, *1965*). Samples of fibronectin were heated at 60°C in Buffer A and examined by the above procedure. After 12 minutes at 60°C pronounced changes were evident in the patterns on the stained gels, indicating aggregation and unfolding of the protein. In contrast, fibronectin pasteurized as above in 57% sucrose for 10 hours showed only a single band, as did unheated fibronectin. The relative mobility of both samples was the same. These results demonstrate that fibronectin does not aggregate or show other structural changes during pasteurization in sucrose.

Immunological tests by the method of quantitative microcomplement fixation (Arnheim, *et al., 1967, J. Biol. Chem.*, Vol. 242, p. 3951). For each sample of fibronectin a reaction curve with specific antiserum was determined. The reaction tubes contained 0.5 ml of diluted antiserum, 0.5 ml of antigen 3-1000 ng of fibronectin), and 0.5 ml of guinea pig complement. Antiserum to fibronectin was diluted 1 : 6000. Results of microcomplement fixation tests were expressed as a percent of the reactivity of unheated purified fibronectin.

Fibronectin pasteurized for 10 hours at 60°C in 54% sucrose showed a 7-12% weaker reaction with antiserum than did unheated fibronectin. For this sensitive test such a variation in cross-reactivity is indicative of little or no change. For example, when unheated fibronectin was compared with human plasma (containing native fibronectin) a 17-30% change was noted. In other protein systems changes of this magnitude are observed between pairs of proteins which differ by single amino acid substitutions (Reichlin, *J. Mol. Biol.*, Vol. 64, p. 485, *1972*) or very slightly in conformation (Prager, *et al., Immunochemistry*, Vol. 3, p. 831, *1974*). These results demonstrate that the structure of fibronectin essentially was unaltered by sucrose pasteurization.

Example 4

Pasteurization of antihemophilic factor

Two vials each from three lots (Lots A, B, and C) of Koate® antihemophilic factor were reconstituted in 10 ml of water-for-injection (WFI) each (approximately 20 mg of protein/ml). The vials were pooled and treated with sucrose at 0.8 g/ml (54%) or 1.2 g/ml (70%). Insolubles were removed by centrifugation. The mixtures were then heated at 60°C for 10 hours to pasteurize them.

As a control, reconstituted antihemophilic factor was treated with sucrose at 0.8 g/ml or 1.2 g/ml. The control and the pasteurized samples from above were assayed according to the method of Example 1. The results are summarized in Table 4 below.

TABLE 4

| | Recovery of VIII:C (%) Amount of sucrose (g/ml) | |
| Lot | 0.8 | 1.2 |
| --- | --- | --- |
| A | 51.4 | 61.7 |
| B | 38.5 | 69.4 |
| C | 23.8 | 66.0 |
| Average | 37.9 | 65.7 |

Example 5

Effect of fibrinogen concentration on pasteurization of AHF

To four final container lots (A-D) of Koate® AHF and one in-process lot (E) was added enough sucrose to make the concentration therein about 54-56%. The fibrinogen content of each lot was determined by RID and cellulose acetate electrophoresis (CAE) ; the RID method gives the amount of fibrinogen in the solution whereas the CAE method gives the amount of fibrinogen in the protein composition. The lots were heated at 60°C for 10 hours ; the results are outlined in Table 5.

TABLE 5

| Lot | Fibrinogen by CAE (%) | Fibrinogen by RID (%) | Observations |
|---|---|---|---|
| A | 41 | 568 | No gelation |
| B | 35 | 194 | No gelation |
| C | 37 | 190 | No gelation |
| D | 62 | 477 | Gelation |
| E | 66 | 940 | Gelation |

## Claims

1. A method for pasteurizing a composition containing a thermally sensitive, therapeutically active protein selected from antithrombin-III, prekallikrein, antihemophilic factor (factor VIII) and fibronectin capable of being stabilized during pasteurization in the presence of a polyol as the only stabilizer which is a water miscible polyhydric alcohol or carbohydrate having a molecular weight less than about 5000, which comprises
   a) mixing about one part of protein composition with 4 to 200 parts of an aqueous polyol having a concentration of said polyol ranging from 30% (w/v) to saturation and
   b) heating the mixture at a temperature of 60 to 75°C at a pH of 5.5 to 8.0 for about 10 hours.

## Ansprüche

1. Verfahren zum Pasteurisieren einer Zusammensetzung, welche ein wärmeempfindliches, therapeutisch aktives Protein, ausgewählt aus Antithrombin-III, Prekallikrein, antihämophilem Faktor (Faktor VIII) und Fibronectin, welches in der Lage ist, während der Pasteurisierung in Gegenwart eines Polyols als einzigem Stabilisator, welches ein wassermischbarer, mehrwertiger Alkohol oder ein Kohlehydrat mit einem Molekurgewicht von weniger als etwa 5.000 ist, stabilisiert zu werden, enthält, umfassend :
   (a) Mischen von etwa 1 Teil der Proteinzusammensetzung mit 4 bis 200 Teilen eines wässrigen Polyols, wobei die Konzentration des Polyols im Bereich von 30% (G/V) bis zur Sättigung liegt, und
   (b) Erwärmen der Mischung auf eine Temperatur von 60 bis 75°C bei einem pH-Wert von 5,5 bis 8,0 während etwa 10 Stunden.

## Revendications

1. Procédé de pasteurisation d'une composition contenant une protéine thermosensible, thérapeutiquement active, choisie entre l'antithrombine III, la prékallikréine, le facteur antihémophilique (facteur VIII) et la fibronectine, capable d'être stabilisée au cours de la pasteurisation en présence d'un polyol comme unique agent stabilisant qui est un alcool polyhydroxylique ou un glucide miscible à l'eau ayant un poids moléculaire inférieur à environ 5000, procédé qui consiste,
   a) à mélanger environ une partie de composition de protéine avec 4 à 200 parties d'un polyol aqueux ayant une concentration en ledit polyol allant de 30% (poids/volume) à la saturation, et
   b) à chauffer le mélange à une température de 60 à 75°C à un pH de 5,5 à 8,0 pendant environ 10 heures.